# EUROPEAN PATENT SPECIFICATION

(11) **EP 3 124 591 B1**
(45) Date of publication and mention of the grant of the patent: **24.10.2018**
(21) Application number: 15838513.8
(22) Date of filing: 10.06.2015
(51) Int. Cl.: C12M 1/00, C12M 3/00, C12M 1/32

(54) **CULTURE VESSEL**
KULTURGEFÄSS
RÉCIPIENT DE CULTURE

(30) Priority: 05.09.2014 JP 2014181187
(43) Date of publication of application: 01.02.2017
(73) Proprietor: Nissha Co., Ltd., Kyoto-shi, Kyoto 604-8551 (JP)
(72) Inventor: OBI, Naoko, Kyoto-shi Kyoto 604-8551 (JP); ONISHI, Atsushi, Kyoto-shi Kyoto 604-8551 (JP); YAMAGUCHI, Yoichi, Kyoto-shi Kyoto 604-8551 (JP); YAMAMOTO, Hiromi, Kyoto-shi Kyoto 604-8551 (JP); NAKAYAMA, Tetsuya, Kyoto-shi Kyoto 604-8551 (JP)
(74) Representative: Kastel, Stefan
(86) International application number: PCT/JP2015/066713
(87) International publication number: WO 2016/035407

(56) References cited:
- EP-A1- 2 230 014
- JP-A- 2003 180 335
- JP-A- 2003 180 335
- JP-A- 2009 050 194
- JP-A- 2010 158 214
- JP-A- 2012 060 903
- US-A- 3 767 790

## Description

### TECHNICAL FIELD

The present invention relates to a culture container, particularly a culture container that contains culture solution for forming spheroids.

### BACKGROUND ART

Cultures of cells, bacteria, and the like are widely performed in the fields of medicine and biology.

In addition, to cultivate a culture using a container suited to the purpose, culture containers of various shapes and sizes have been developed.

Particularly in recent years, unlike conventional planar cell cultures, attention has been drawn to cell spheroids (hereinbelow, called spheroids) prepared by culturing in three dimensions with the aim of causing the cells to sufficiently exhibit their intrinsic functions. Spheroids are three-dimensional structural bodies formed by adhering the cells to one another utilizing a cell-adhering function possessed by the cells.

One known method of forming spheroids is a technique that injects a suspension of cells into a culture container, which has a plurality of recessed parts, and forms the spheroids in the recessed parts (e.g., refer to Patent Citations 1, 2, 3, and 4).

### CITATION LIST

### PATENT CITATIONS

Patent Citation 1: JP 2010-88347 A
Patent Citation 2: EP 2 230 014 A1
Patent Citation 3: US 3 767 790 A
Patent Citation 4: JP 2003-180335 A

### SUMMARY OF INVENTION

### TECHNICAL PROBLEM

In the cell-culture container described in Patent Citation 1, the spheroids are formed in a state wherein the cells are suspended in the suspension. In this method, there are cases in which the spheroids flow out of the recessed parts owing to the convection of the suspension. The convection of the suspension arises due to: vibration that occurs when, for example, the container is carried to a microscope in order for the spheroids to be observed; the suspension is moving in and out in order to be replaced; and the like. Spheroids that flow out of the recessed parts adversely adhere to one another, and consequently there are cases in which the sizes of the spheroids become uneven. In addition, there are cases in which the spheroids that flow out of the recessed parts are adversely sucked in the exterior together with the suspension.
According to Patent Citation 2, a culture container for conducting a method to reproducibly and efficiently generate cell aggregates is provided having a microwell plate at the bottom region and a particle retaining member.

An object of the present invention is to enhance a stable formation of spheroids in a culture container.

### TECHNICAL SOLUTION

This object is solved by a culture container according to claim 1. Advantageous embodiments of the invention are indicated in the dependent claims.

Some advantageous aspects of embodiments of the present invention are explained below as the technical solution. These aspects can be arbitrarily combined as needed.

A culture container according to one aspect of the present invention includes a container main body and a filmlike member. The container main body has a recessed part, wherein a plurality of microwells for containing culture solution is formed on a bottom surface. The filmlike member is a member that is disposed above the plurality of microwells inside the recessed part. The filmlike member limits movement of spheroids grown inside the microwells so that the spheroids do not separate from the microwells.

In the present culture container, each filmlike member is disposed above the plurality of microwells inside the corresponding recessed part and limits the movement of the spheroids grown inside those microwells such that the spheroids do not separate from those microwells. Accordingly, the spheroids are formed stably in the culture container.

The filmlike member may have a shape or a property such that the culture solution can pass through.

In this case, the culture solution in the plurality of microwells can be replaced easily.

The filmlike member may have a mesh structure.

In this case, the culture solution can pass through any location of the filmlike member, and therefore the culture solution inside the plurality of microwells can be replaced efficiently.

A lower surface of the filmlike member may be subject to a cell nonadherence treatment.

In this case, the lower surface of the filmlike member is treated such that the spheroids tend not to adhere thereto; as a result, the spheroids tend not to adhere to the lower surface of the filmlike member.

The filmlike member may be soluble.

In this case, after the spheroids have been formed, the filmlike member dissolves and disappears. Consequently, a procedure to remove the filmlike member becomes unnecessary. That is, the removal of the spheroids becomes easy.

The culture container may includes a tubular member capable of being inserted into the recessed part and having a bottom part at which the filmlike member is provided.

In this case, the filmlike member can be installed and removed by the insertion and removal of the tubular member into and from the recessed part. As a result, work efficiency is improved.

A gap is ensured between the bottom surface of the recessed part and a lower surface of the filmlike member, and thereby the culture solution can move in and out of each microwell.

In this case, the culture solution inside the plurality of microwells can be replaced even in a case wherein a filmlike member through which the culture solution can pass is not used.

### ADVANTAGEOUS EFFECTS

In a culture container according to the present invention, spheroids are stably formed.

### BRIEF DESCRIPTION OF DRAWINGS

Only figures 22-25 correspond to embodiments of the claimed invention.
FIG.1 is an oblique view of a container main body of a cell-culture container.
FIG. 2 is an oblique view of a presser-member-attached plate of the cell-culture container.
FIG. 3 is an oblique view of the presser-member-attached plate of the cell-culture container.
FIG. 4 is a cross-sectional view of the cell-culture container.
FIG. 5 is a cross-sectional view of the cell-culture container.
FIG. 6 is a plan view of a filmlike member.
FIG. 7 is a schematic drawing for explaining spheroid preparation.
FIG. 8 is a schematic drawing for explaining spheroid preparation.
FIG. 9 is a schematic drawing for explaining spheroid preparation.
FIG. 10 is a plan view of the filmlike member (second embodiment).
FIG. 11 is a plan view of the filmlike member (third embodiment).
FIG. 12 is a plan view of the filmlike member (fourth embodiment).
FIG. 13 is a cross-sectional view of the cell-culture container (fifth embodiment).
FIG. 14 is a cross-sectional view of the cell-culture container (sixth embodiment).
FIG. 15 is a cross-sectional view of the cell-culture container (seventh embodiment).
FIG. 16 is a cross-sectional view of the cell-culture container.
FIG. 17 is a cross-sectional view of the cell-culture container.
FIG. 18 is a cross-sectional view of the cell-culture container.
FIG. 19 is a top view of a presser member.
FIG. 20 is a top view of the presser member (eighth embodiment).
FIG. 21 is a cross-sectional view of the cell-culture container (ninth embodiment).
FIG. 22 is a cross-sectional view of the cell-culture container (tenth embodiment).
FIG. 23 is a cross-sectional view of the cell-culture container (eleventh embodiment).
FIG. 24 is a cross-sectional view of the cell-culture container (thirteenth embodiment).
FIG. 25 is a cross-sectional view of the cell-culture container (fourteenth embodiment).
FIG. 26 is an oblique view of the container main body of the cell-culture container (fifteenth embodiment).

### DESCRIPTION OF EMBODIMENTS

### 1. First Embodiment (not part of the invention)

### (1) Overall Structure

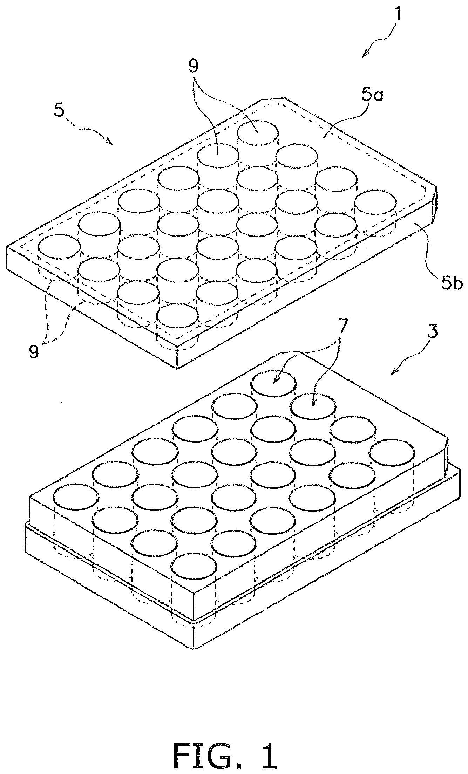

A cell-culture container 1, which serves as a culture container according to one embodiment of the present invention, will now be explained, with reference to FIG. 1 to FIG. 3.

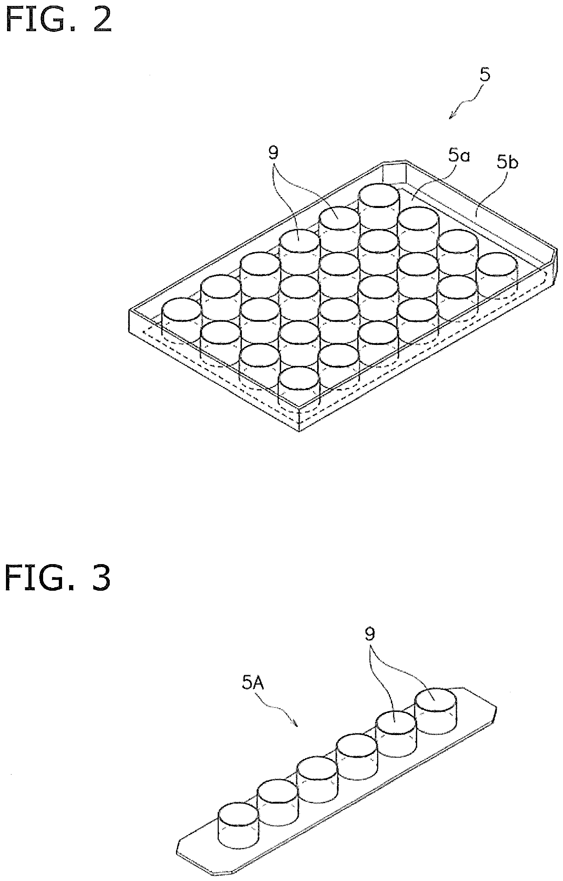

FIG. 1 is an oblique view of a container main body of a cell-culture container. FIG. 2 and FIG. 3 are oblique views of a presser-member-attached plate of the cell-culture container.

As shown in the figures, the cell-culture container 1 includes a container main body 3 and a presser-member-attached plate 5. The container main body 3 has an oblong shape in plan view and includes a plurality of recessed parts 7 (wells) that serves as cell-culture cells for containing cell-culture solution C, which serve as culture solution. The container main body 3 is a transparent member having a thin wall thickness and is made of, for example, a transparent plastic. The container main body 3 is one that is well known and may be, for example, a general well plate.

The presser-member-attached plate 5 has an oblong shape that corresponds to the container main body 3 and includes a plurality of presser members 9 corresponding to the recessed parts 7. The presser-member-attached plate 5 is a transparent member having a thin wall thickness and is made of, for example, a transparent plastic. The presser-member-attached plate 5 includes a plate-shaped main body 5a and a frame 5b, which is formed on an outer-perimetric surface of the main body 5a. The plurality of presser members 9 are provided on the main body 5a of the presser-member-attached plate 5. The presser members 9 are members that are removably fitted into upper sides of the recessed parts 7.

Furthermore, the presser-member-attached plate 5 shown in FIG. 2 includes 6 × 4, that is, a total of 24, of the presser members 9, and a presser-member-attached plate 5A shown in FIG. 3 includes 6 × 1, that is, a total of six, of the presser members 9. Four of the presser-member-attached plates 5A shown in FIG. 3 may be used for the container main body 3 shown in FIG. 1.

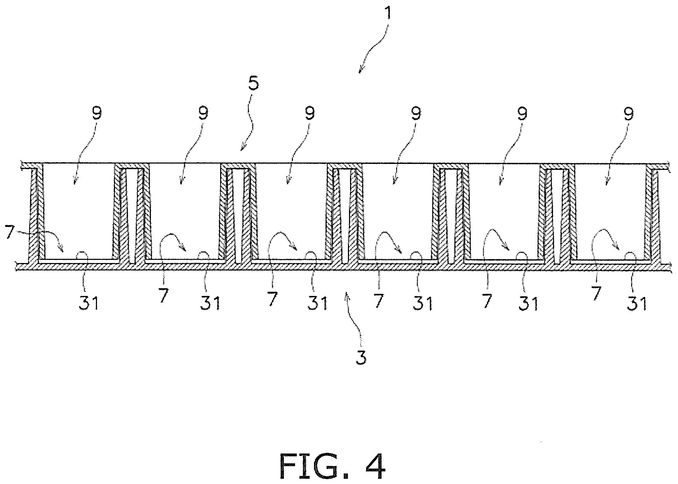

FIG. 4 is a cross-sectional view of the cell-culture container. As shown in FIG. 4, in the state wherein the presser-member-attached plate 5 is fitted to the container main body 3, the plurality of presser members 9 are inserted into the plurality of recessed parts 7.

Furthermore, although not shown, during cell culturing, the cell-culture container 1 is covered with a cover from above (that is, from above the presser-member-attached plate 5). The cover covers the container main body 3 and an upper part of the presser-member-attached plate 5. Thereby, contamination of the cell-culture solution is prevented.

### (2) Detailed Structure

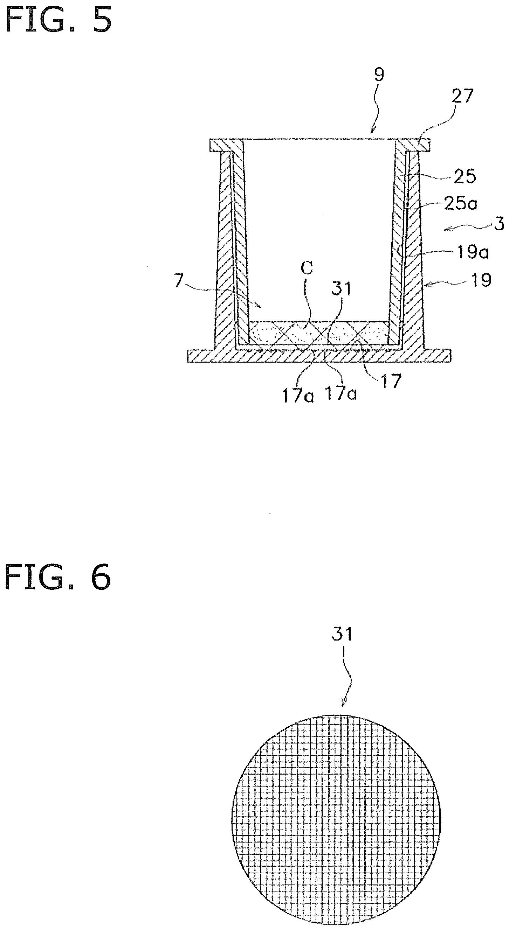

Next, the structures of the recessed parts 7 and the presser members 9 and the relationship of the two will now be explained in detail, with reference to FIG. 5 and FIG. 6. FIG. 5 is a cross-sectional view of the cell-culture container, and FIG. 6 is a plan view of a filmlike member.

The recessed part 7 includes a bottom part 17 and a tubular part 19. An inner circumferential surface 19a of the tubular part 19 extends substantially vertically in a longitudinal cross section. An upper surface of the bottom part 17 is non-adherent with respect to cells. Cell nonadherency refers to a material property in which adherent cells tend not to adhere to the material. To achieve cell nonadherency, for example, polyethylene glycol, polyhydroxyethyl methacrylate, or ethylene vinyl alcohol copolymer may be used as, for example, a substance having high hydrophilicity. In addition, to impart hydrophilicity to the upper surface of the bottom part 17, the upper surface may be coated with a surfactant, phospholipids, or the like, or may be subject to a surface treatment such as a plasma treatment.

A plurality of microwells 17a is formed on the upper surface of the bottom part 17. The microwells 17a are micro-recessed parts for forming spheroids. The diameter of each microwell 17a is, for example, 30-1,500 µm and preferably is 50-300 µm. The depth of each microwell 17a is, for example, 30-1,500 µm and preferably is 50-300 µm.

The presser member 9 principally includes a tubular part 25 and a filmlike member 31. The tubular part 25 is fitted, with a slight gap, into the tubular part 19 of the recessed part 7. That is, an outer circumferential surface 25a of the tubular part 25 opposes the inner circumferential surface 19a of the tubular part 19 in a state wherein the outer circumferential surface 25a is proximate to the inner circumferential surface 19a. An outer circumferential edge of the filmlike member 31 is fixed to a lower end of the tubular part 25. The filmlike member 31 may be formed integrally with the tubular part 25.

The filmlike member 31 is a member that is disposed above the microwells 17a inside the recessed part 7 and is for limiting movement of the objects that have grown inside the microwells 17a such that those objects do not separate from the microwells 17a. In the present embodiment, the filmlike member 31 has a shape or a property through which the cell-culture solution can pass. Specifically, as shown in FIG. 6, the filmlike member 31 may be a circular mesh member. Because the filmlike member 31 is a mesh member, the cell-culture solution can move, over the entirety of the mesh member, across both sides.

In the present embodiment, a lower surface of the filmlike member 31 is subject to a cell nonadherence treatment. The cell nonadherence treatment is, for example, the same as the one discussed above. Owing to this treatment, spheroids S tend not to adhere to the filmlike member 31, as discussed below. Furthermore, the cell nonadherence treatment does not necessarily have to be performed.

In the present embodiment, the filmlike member 31 is soluble. The filmlike member 31 dissolves in, for example, one week. The filmlike member 31 is, for example, crosslinked gelatin, starch, or PVA. As a result, as described below, the spheroids S become easy to remove. Furthermore, the filmlike member 31 does not necessarily have to be soluble. In such a case, the filmlike member 31 may be, for example, nylon, PE, PP, carbon, PEEK, Teflon®, PET, or a metal.

The dimension of the mesh opening of the filmlike member 31 is, for example, 30-1,500 µm. The dimension between the filmlike member 31 and the upper surface of the bottom part 17 is, for example, 50-300 µm. Furthermore, the dimension of the spheroid is 30-1,500 µm, and the dimension of the mesh opening is adjusted to match that size.

Furthermore, in the abovementioned embodiment, the dimension of the mesh opening is set such that seed cells c can pass through, but it may be set such that the seed cells c cannot pass through. In such a case, the seed cells c are seeded in the microwells 17a prior to the fixing of the filmlike member 31.

The presser member 9 further includes a flange part 27. In the present embodiment, for the sake of convenience of the explanation, the flange part 27 is explained as a member that is fixed to the main body 5a of the presser-member-attached plate 5, but the flange part 27 may be integrally formed with the main body 5a. The flange part 27 extends radially outward of the tubular part 25 and is seated on the upper surface of the tubular part 19 of the recessed part 7. Thus, because the presser member 9 is provided with the flange part 27, it is easy to remove the presser member 9 from the recessed part 7.

### (3) Spheroid-Preparing Process

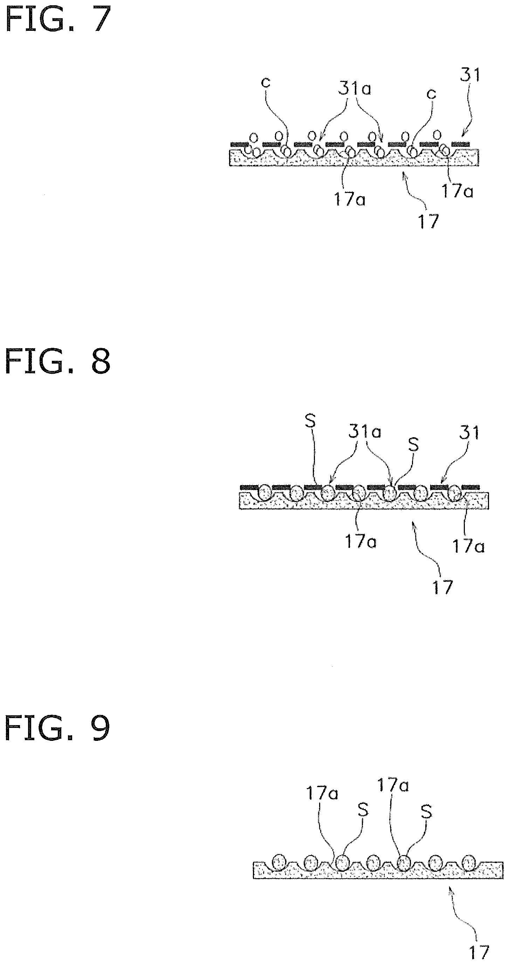

A process of forming the spheroids S will now be explained, with reference to FIG. 7 to FIG. 9. FIG. 7 to FIG. 9 are schematic drawings for explaining spheroid preparation.

First, the seed cells c are seeded. Specifically, as shown in FIG. 7, the cell-culture solution is injected into the recessed part 7. The cell-culture solution is a suspension including a liquid culture medium and the seed cells c, which are evenly dispersed in the culture medium. The seed cells c are adherent, for example: cancer cells, such as human osteosarcoma cells; hepatocytes; and the like. A well-known culture medium suited to the culturing of adherent cells is used as the culture medium. In the above case, the seed cells c are implanted by dropping them as far as the microwells 17a through gaps 31a of the filmlike member 31.

Furthermore, as shown in FIG. 8, the spheroids S are formed by the agglomeration of the plurality of seed cells c inside each microwell 17a. In the present cell-culture container 1, the filmlike member 31 is disposed above the plurality of microwells 17a inside the recessed part 7, and the movement of each spheroid S that has grown inside its corresponding microwell 17a is limited such that each spheroid S does not separate from the corresponding microwell 17a. Accordingly, the spheroids S are formed stably in the cell-culture container 1.

An apparatus that supplies and discharges the culture medium to and from the interior of the recessed part 7 may be provided using a pipe and a pump, which are not shown in the figures. Thereby, new culture medium can be injected into the recessed part 7 through an inflow port, and culture medium inside the spheroid-culture container can be discharged through an outflow port. That is, when the seed cells c are being formed into the spheroids S in the microwells 17a, the culture medium inside the recessed part 7 may be replaced.

In the abovementioned replacement of the culture medium, a flow occurs in the culture medium inside the recessed part 7; however, at this time, the spheroids S do not flow out of the microwells 17a, because the filmlike member 31 is pressed against the spheroids S. Accordingly, the outflow of the spheroids S is prevented.

Owing to the culture medium replacement discussed above, the circulation of the culture medium supplies nutrients and oxygen to the cells even while the cells are being cultured, and therefore the cells grow healthfully. As a result, large spheroids S that require a relatively long culture time can be formed; furthermore, the formed spheroids S can be preserved for a long time. In addition, owing to the culture medium replacement, cell waste and waste matter that did not form into spheroids S are removed.

Furthermore, as shown in FIG. 9, after the spheroids S have been formed, the filmlike member 31 dissolves and thereby the spheroids S are exposed. As a result, it becomes easy to remove the spheroids S. Furthermore, if the filmlike member 31 is not soluble, then the state in FIG. 9 is obtained by the removal of the filmlike member.

### 2. Second Embodiment (not part of the invention)

The filmlike member is not limited to a mesh member as long as it has a shape through which the cell-culture solution can pass.

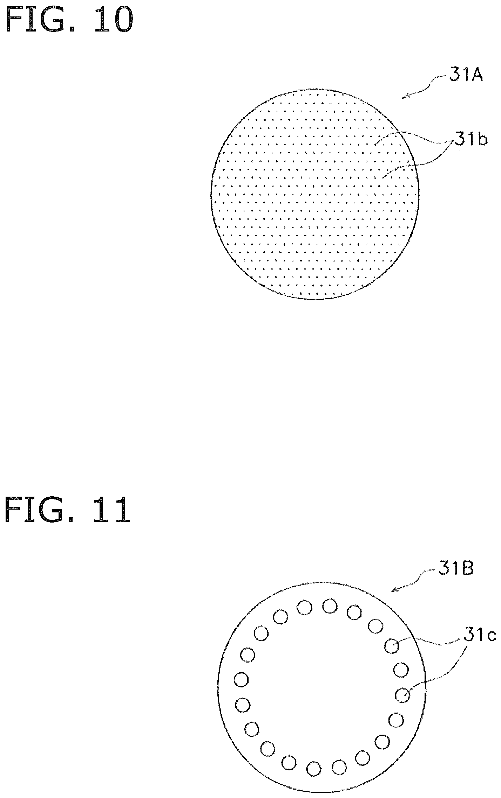

As shown in FIG. 10, a filmlike member 31A is a circular film in which a plurality of micropores 31b is formed over the entirety.

The filmlike member 31A may be, for example, glass, a plastic film, or a plastic plate. The plurality of micropores 31b is formed by, for example, a laser.

### 3. Third Embodiment (not part of the invention)

The filmlike member is not limited to a mesh member as long as it has a shape through which the cell-culture solution can pass.

As shown in FIG. 11, a filmlike member 31B is a circular member in which a plurality of small holes 31c is formed. The filmlike member 31B is a material that is the same as that of the filmlike member 31A.

In the present embodiment, the plurality of small holes 31c is disposed in a ring shape on the outer-circumference side. The shape, positions, and number of the holes are not particularly limited. However, if the plurality of small holes is formed only on the outer-circumference side of the filmlike member, as in FIG. 11, then the accuracy with which the spheroids S are observed in the microwells corresponding to the center part and the midway part of the filmlike member is improved.

### 4. Fourth Embodiment (not part of the invention)

The filmlike member 31 is not limited to those in the first embodiment and the second embodiment, as long as it has a property through which the cell-culture solution can pass.

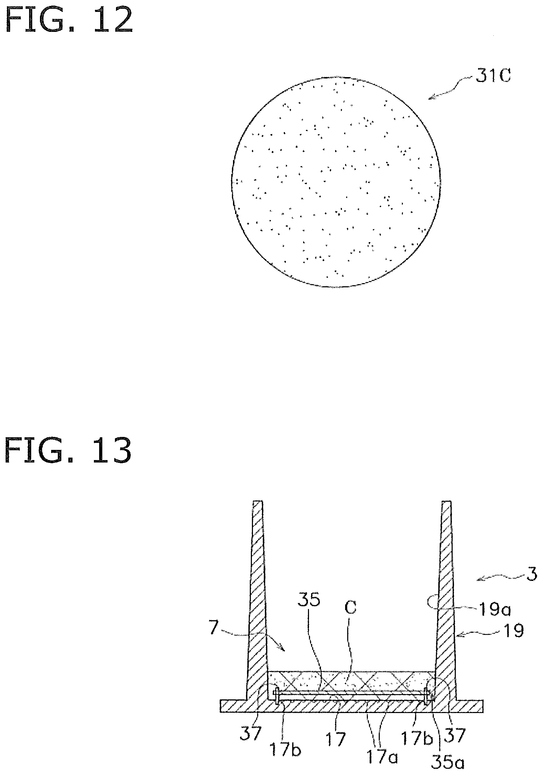

As shown in FIG. 12, a filmlike member 31C includes a hydrogel. The hydrogel is a macromolecular material having a property by which the material swells due to the inclusion of a large amount of moisture. The hydrogel may be, for example, acrylamide, silicone, agarose, or gelatin. Thereby, the cell-culture solution can pass through the filmlike member 31C. Furthermore, in the case of hydrogel, the structure should be capable of circulating protein (approximately 1-20 nm).

In this case, the seeding of the cells is performed prior to the installation of the filmlike member 31C. This is because, although the culture medium passes through the filmlike member 31C, the cells do not pass through.

Furthermore, the filmlike member is not limited to hydrogel, as long as the material has a property such that the cell-culture solution can pass through.

### 5. Fifth Embodiment (not part of the invention)

In the first through fourth embodiments, the filmlike member has a shape or a property such that the cell-culture solution can pass through, but the filmlike member is not limited to those described above as long as it is capable of limiting movement such that objects grown inside the microwells do not depart from the microwells. For example, the filmlike member may have a shape or a property such that the cell-culture solution cannot pass through.

The details of the above will now be explained, with reference to FIG. 13. FIG. 13 is a cross-sectional view of the cell-culture container.

As shown in the figure, the filmlike member 35 is disposed at a position proximate to the upper surface of the bottom part 17 of the recessed part 7. The filmlike member 35 has a property such that the cell-culture solution cannot pass through. A ring-shaped gap 35a is ensured between the outer circumferential edge of the filmlike member 35 and a lower part of the inner circumferential surface 19a of the tubular part 19.

The gap between the filmlike member 35 and the upper surface of the bottom part 17 of the recessed part 7 has a dimension such that one cell can pass through but a spheroid cannot pass through. For example, the gap is greater than or equal to 10 µm and less than or equal to 100 µm. Furthermore, the dimension of the spheroid is 30-1,500 µm and the dimension of the gap is adjusted to match that size.

The filmlike member 35 is fixed to the bottom part 17 by a plurality of fixing pins 37. The fixing pins 37 are mounted to an outer-circumference part of the filmlike member 35, and tips of the fixing pins 37 are inserted into mounting holes 17b of the bottom part 17.

The filmlike member 35 is a transparent film. The filmlike member 35 may be, for example, nylon, PE, PP, carbon, PEEK, Teflon ®, or PET.

In the present embodiment, at the outer-circumference part of the filmlike member 35, the cell-culture solution can move, through the gaps 35a, alternately in and out of a space between the filmlike member 35 and the bottom part 17 (that is, the space in which the spheroids S are formed in the microwells 17a). That is, it is possible to replace the culture medium while the filmlike member 35 is pressing against the spheroids S. Owing to the culture medium replacement, the cells are supplied with nutrients and oxygen even while being cultured, and therefore the cells grow healthfully. In addition, owing to the culture medium replacement, cell waste and waste matter that did not form into spheroids S are removed.

In the present embodiment, effects the same as those in the first through fourth embodiments are obtained. In addition, modified examples of each embodiment also can be adapted to the present embodiment where appropriate.

Furthermore, because the filmlike member 35 is transparent, the spheroids S can be observed with a microscope.

### 6. Sixth Embodiment (not part of the invention)

The structure by which the filmlike member is attached in the vicinity of the bottom part is not limited to that of the fifth embodiment.

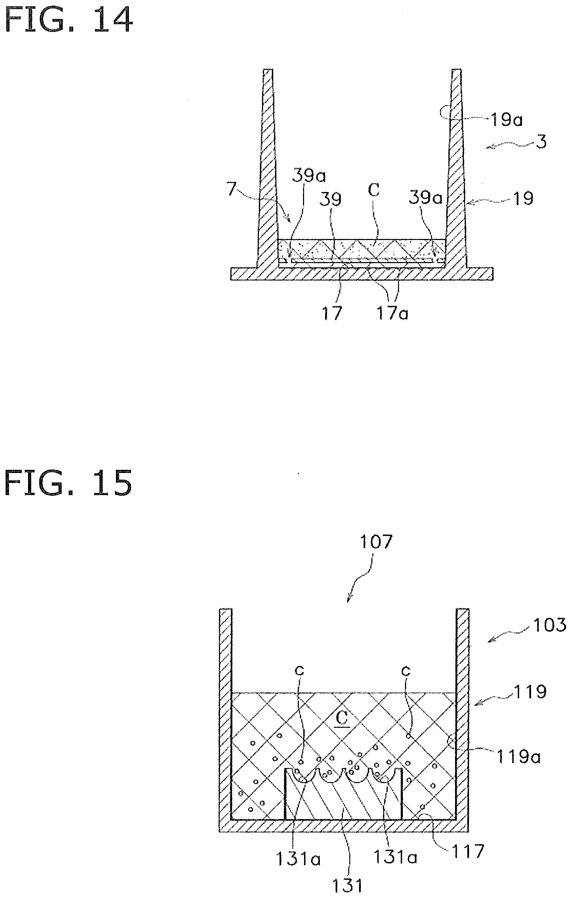

Such an embodiment will now be explained, with reference to FIG. 14. FIG. 14 is a cross-sectional view of the cell-culture container.

As shown in the figure, a filmlike member 39 is disposed at a position proximate to the upper surface of the bottom part 17 of the recessed part 7. The filmlike member 39 has a shape or a property such that the culture solution cannot pass through.

The outer circumferential edge of the filmlike member 39 is fixed to the lower part of the inner circumferential surface 19a of the tubular part 19. In addition, a plurality of holes 39a is formed in the outer circumference of the filmlike member 39.

In the present embodiment, at the outer-circumference part of the filmlike member 35, the cell-culture solution can move, through the plurality of holes 39a, alternately in and out of the space between the filmlike member 35 and the bottom part 17 (that is, the space in which the spheroids S are formed in the microwells 17a).

### 7. Seventh Embodiment (not part of the invention)

The first through sixth embodiments use a container wherein the microwells are formed at the bottom part, but the member wherein the microwells are formed is not limited to the abovementioned configuration.

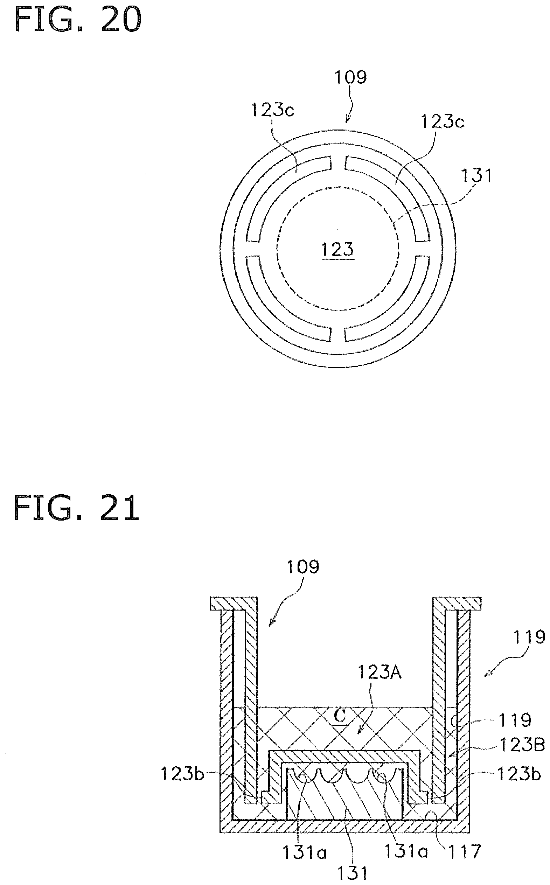

A cell-culture container for forming spheroids will now be explained, with reference to FIG. 15 to FIG. 19. FIG. 15 to FIG. 19 are cross-sectional views of the cell-culture container. FIG. 20 is a top view of the presser member.

FIG. 15 shows a container main body 103 of the cell-culture container. The container main body 103 is a container that has a recessed part 107, the upper side of which is open, and holds the cell-culture solution C. The cell-culture solution C contains a large number of the seed cells c.

A microwell member 131 is installed on a bottom part 117 of the recessed part 107. The microwell member 131 is a member having a plurality of microwells 131a on its upper surface.

In the present embodiment, the microwell member 131 is provided at the center of the bottom part 117, and the upper surface of the microwell member 131 is disposed relatively spaced apart and upward from the bottom part 117. Consequently, in the bottom part 117, a portion radially outward of the microwell member 131 is a surface that is lower than the upper surface of the microwell member 131.

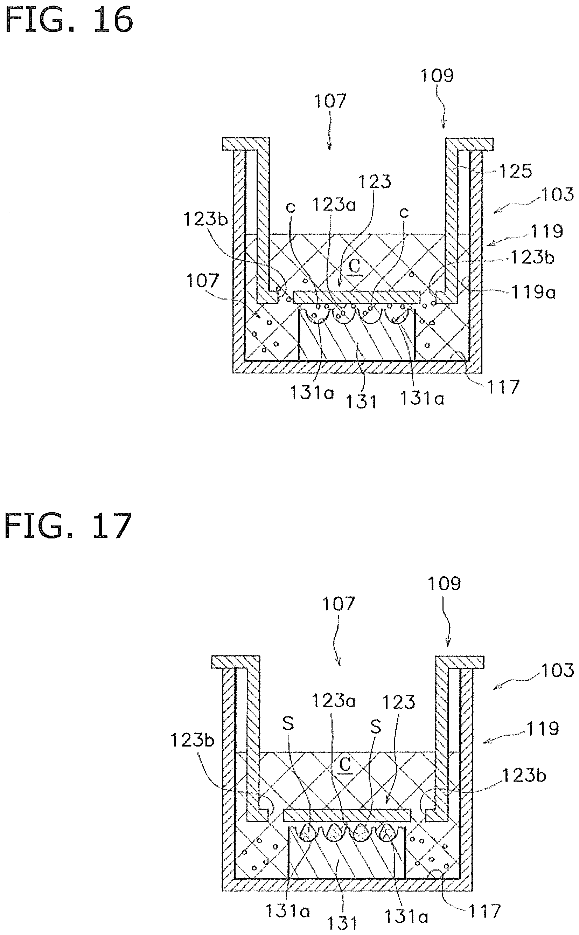

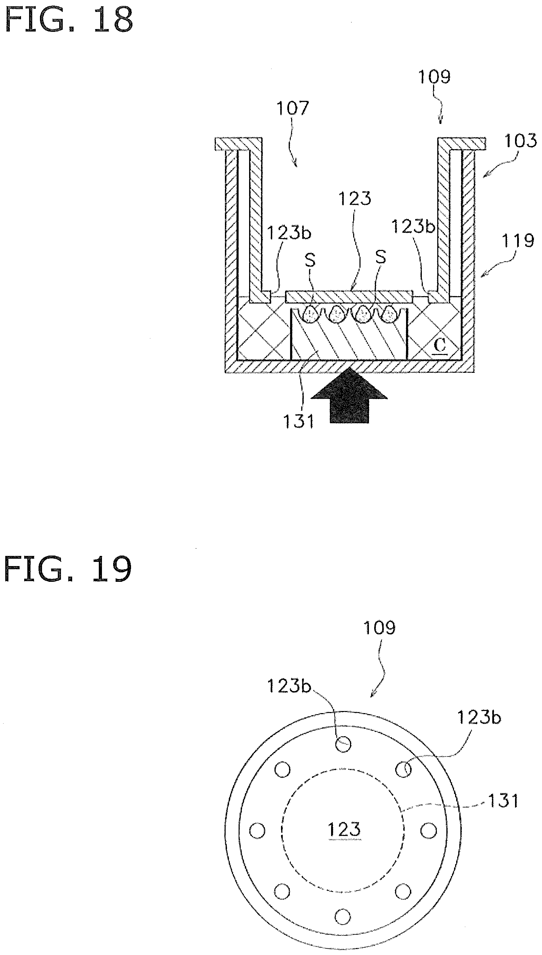

As shown in FIG. 16, a presser member 109 is inserted into the recessed part 107 of the container main body 103. As shown in FIG. 16 and FIG. 19, the presser member 109 has a discoidal lower-surface part 123 that serves as the filmlike member. The lower-surface part 123 is disposed proximate to the upper surface of the microwell member 131 (the portion at which the microwells 131a are formed). In addition, in the lower-surface part 123, a plurality of bubble-discharge parts 123b is formed in a portion radially outward from the microwell member 131. That is, the bubble-discharge parts 123b are provided so as to correspond to the portion of the bottom part 117 at which the plurality of microwells 131a are not formed. The bubble-discharge parts 123b are dotlike through holes. Accordingly, bubbles in the space below the lower-surface part 123 (e.g., the plurality of microwells 131a and the periphery of the microwell member 131) are easily discharged to the exterior.

When cells are cultured in the abovementioned state, the spheroids S are formed inside the microwells 131a, as shown in FIG. 17. Here, because the lower-surface part 123 is disposed proximate to the microwells 131a, the spheroids S grown do not go out of the microwells 131a. Accordingly, the spheroids S can be formed safely and reliably.

As shown in FIG. 18, after the amount of the cell-culture solution C has decreased and the cell-culture solution C no longer exists on the upper side of the lower-surface part 123, the spheroids S are observed from below the container main body 103. At this time, a lower surface 123a of the lower-surface part 123 makes contact with the upper surface of the cell-culture solution C, and therefore the meniscus is eliminated. As a result, the spheroids S in the container main body 103 can be accurately observed.

### 8. Eighth Embodiment (not part of the invention)

In the seventh embodiment, the bubble-discharge parts are a plurality of holes, but the configuration of the bubble-discharge parts is not limited thereto.

Another embodiment of the presser member will be explained, with reference to FIG. 20. FIG. 20 is a top view of the presser member. In the lower-surface part 123, a plurality of bubble-discharge parts 123c is formed in a portion radially outward of the microwell member 131.

The bubble-discharge parts 123c are arcuate through grooves extending in the circumferential direction.

The shape, number, and positions of the through holes formed in the lower-surface part and serving as the bubble-discharge parts are not particularly limited.

In addition, the bubble-discharge parts are not limited to being through holes formed in the lower-surface part. The bubble-discharge parts may be notches, slits, or through holes formed between a tubular part 125 of the presser member 109 and a tubular part 119 of the container main body 103.

### 9. Ninth Embodiment (not part of the invention)

In the seventh embodiment, the lower-surface part of the presser member has a flat shape, but the shape of the lower-surface part is not particularly limited.

Another embodiment of the presser member 109 will now be explained, with reference to FIG. 21. FIG. 21 is a cross-sectional view of the cell-culture container.

As shown in the figure, the presser member 109 includes a flat lower-surface part 123A and a ring-shaped protruding part 123B. The lower-surface part 123A is disposed proximate to the upper surface of the microwell member 131. The ring-shaped protruding part 123B is formed on the outer circumferential edge of the lower-surface part 123A and extends downward. That is, the protruding part 123B is disposed so as to surround the outer-circumference side of the microwell member 131. The plurality of bubble-discharge parts 123b is formed on a bottom surface of the protruding part 123B. The shapes of the bubble-discharge parts 123b are dotlike through holes, arcuate through holes, or a combination thereof.

### 10. Tenth Embodiment (invention)

Particularly in the first through ninth embodiments, it is required to accurately control the dimension between the lower-surface part of the presser member and the members therebelow. However, because the presser member is used by being pressed onto the culture medium in the culture container, there is conceivably a problem in that the presser member will adversely float owing to its buoyancy.

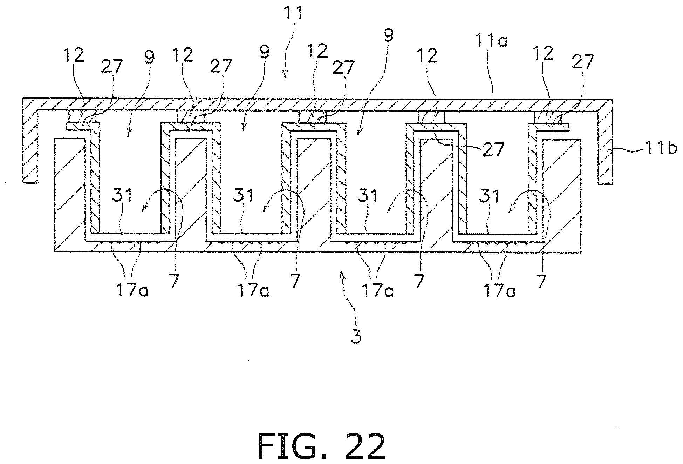

Accordingly, a structure for solving such a problem will now be explained, with reference to FIG. 22. FIG. 22 is a cross-sectional view of the cell-culture container.

As shown in the figure, a cover 11 is disposed above the presser-member-attached plate 5. The cover 11 covers the entire upper side of the presser-member-attached plate 5. The cover 11 includes a flat main body 11a and a tubular part 11b, which extends downward from the outer circumferential edge of the main body 11a. Cushion members 12 are disposed between the main body 11a of the cover 11 and the presser-member-attached plate 5. The cushion members 12 are elastic members such as springs, rubber, and sponges.

The cushion members 12 are compressed between the presser-member-attached plate 5 and the cover 11 and thereby generate an elastic force. Thereby, the presser-member-attached plate 5 is prevented from floating upward and can be fixed inside the container main body 3. Thereby, for example, in a case of forming spheroids, the gap between the microwells and the mesh can be accurately ensured, and thereby a spheroid-fixing effect is reliably obtained.

### 11. Eleventh Embodiment (invention)

In the twelfth embodiment, the elastic members are compressed only by the intrinsic weight of the cover, but the elastic members may be compressed by some other structure.

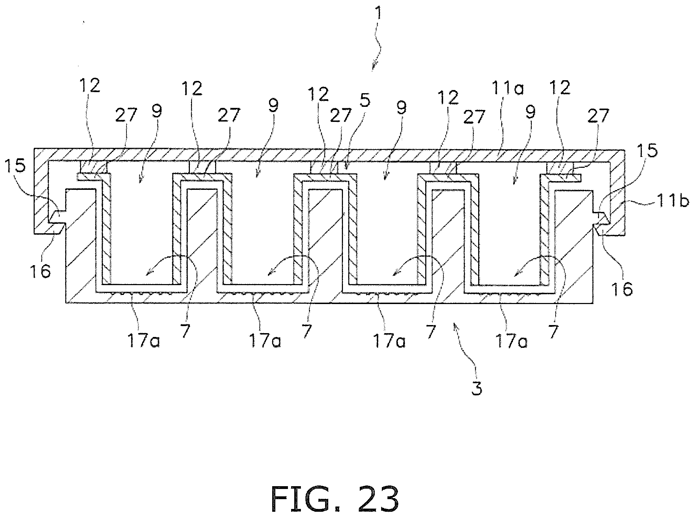

Accordingly, a structure for solving such a problem will now be explained, with reference to FIG. 23. FIG. 23 is a cross-sectional view of the cell-culture container.

The basic structure is the same as in the tenth embodiment.

In the present embodiment, a first engaging part 15 is formed on an outer-circumference part of the container main body 3, and furthermore a second engaging part 16 is formed on the tubular part 11b of the cover 11. The first engaging part 15 and the second engaging part 16 engage with one another, and thereby the cover 11 does not separate from the container main body 3.

In the present embodiment, the cushion members 12 are compressed between the presser-member-attached plate 5 and the cover 11, and thereby the elastic force is generated. Thereby, the presser-member-attached plate 5 is prevented from floating upward and can be fixed inside the container main body 3. Thereby, for example, even in a case of forming spheroids, the gap between the microwells and the mesh can be sufficiently shortened, and thereby a spheroid-fixing effect is reliably obtained.

### 12. Twelfth Embodiment (invention)

In the tenth embodiment and the eleventh embodiment, the presser-member-attached plate 5 is prevented from floating upward by the use of the cover and the cushion members, but it is possible to prevent upward flotation also by other means. For example, the floating due to buoyancy can be prevented by increasing the weight of the presser-member-attached plate 5. Specifically, the intrinsic weight of the presser-member-attached plate 5 can be increased by using a metal material in parts. In addition, a weight can be attached to part of the presser-member-attached plate 5.

### 13. Thirteenth Embodiment (invention)

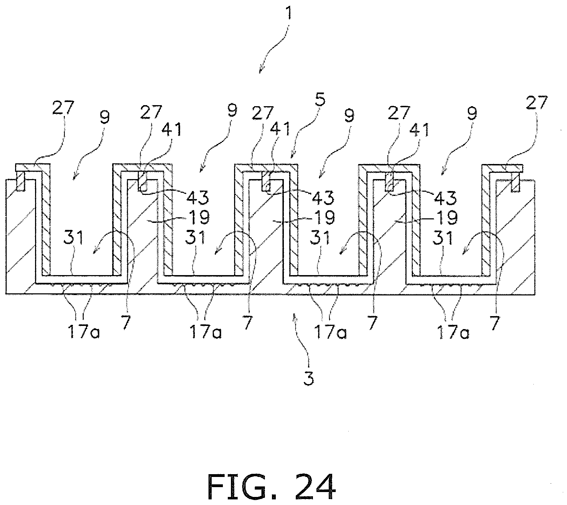

A structure for accurately positioning the filmlike member 31, which is fixed to the tubular part 19, will now be explained, with reference to FIG. 24. FIG. 24 is a cross-sectional view of the cell-culture container.

Pin members 41 are fixed to the lower surfaces of the flange parts 27 of the presser-member-attached plate 5. Lower ends of the pin members 41 are inserted into holes 43, which are formed in the upper surfaces of the tubular parts 19 of the container main body 3.

Thus, the presser members 9 and the recessed parts 7 are accurately positioned in the up-down direction by the pins and the holes. Accordingly, the gaps between the filmlike members 31 and the upper surfaces of the bottom parts 17 are accurately ensured.

Furthermore, the number, shape, and positions of the positioning structures configured by the abovementioned pins and holes are not particularly limited.

### 14. Fourteenth Embodiment (invention)

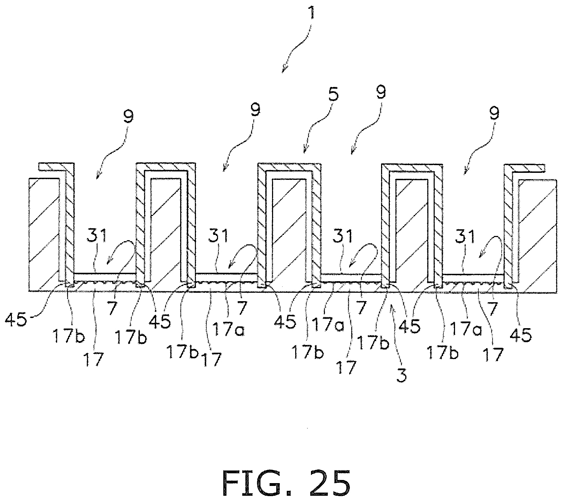

A structure for accurately positioning the filmlike member 31, which is fixed to the tubular part 19, will now be explained, with reference to FIG. 25. FIG. 25 is a cross-sectional view of the cell-culture container.

A plurality of projections 45 is provided on the lower ends of the tubular parts 19. The projections 45 extend further downward of the filmlike members 31. The lower ends of the projections 45 are inserted into the mounting holes 17b, which are formed in the upper surfaces of the bottom parts 17.

Thus, the presser members 9 and the recessed parts 7 are accurately positioned in the up-down direction by the projections and the holes. Accordingly, the gaps between the filmlike members 31 and the upper surfaces of the bottom parts 17 are accurately ensured.

Furthermore, the number, shape, and positions of the positioning structures configured by the abovementioned projections and holes are not particularly limited. In addition, the positioning between the lower parts of the tubular parts and the bottom parts is not limited to the embodiments. For example, the lower parts of the tubular parts may be supported by receiving parts provided on the bottom parts.

### 15. Fifteenth Embodiment (not part of the invention)

The following explains positioning structures provided to both the plurality of presser members and the plurality of recessed parts such that the plurality of presser members fits smoothly into the plurality of the recessed parts.

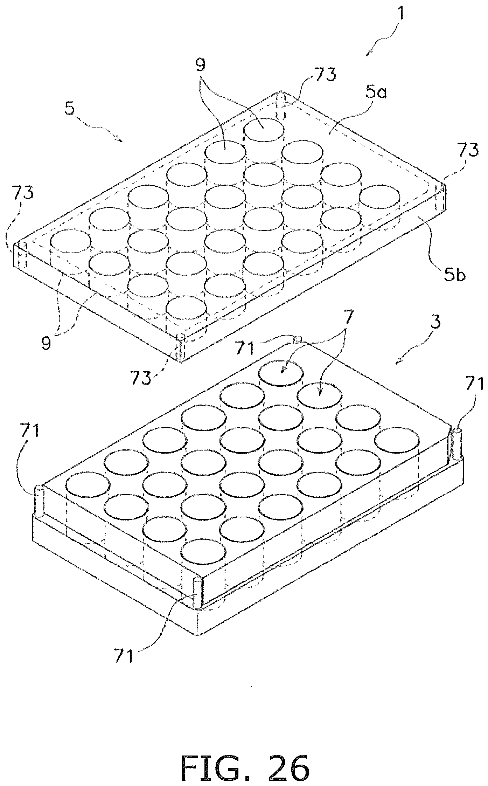

Such an embodiment will now be explained, with reference to FIG. 26. FIG. 26 is an oblique view of the container main body of the cell-culture container.

Furthermore, the basic structure is the same as in the first through fifteenth embodiments. The explanation below focuses on points of difference.

A plurality of pins 71 are uprightly provided at corners on the upper side of the container main body 3. Specifically, the pins 71 are disposed at the four corners of an outer-side portion of a main-body portion wherein the recessed parts 7 of the container main body 3 are formed.

Holes 73 are formed in the presser-member-attached plate 5 at positions corresponding to the pins 71. Specifically, the holes 73 are formed at the four corners of the frame 5b.

When the presser-member-attached plate 5 is being fitted onto the container main body 3, the presser-member-attached plate 5 and the container main body 3 are positioned by the pins 71 and the holes 73, and thereby the plurality of presser members 9 is fitted smoothly into the recessed parts 7.

Furthermore, the number of the pins and the holes is not limited to the embodiments. Furthermore, the positioning structures of the container main body 3 and the presser-member-attached plate 5 are not limited to pins and holes.

### 16. Common Features of the Embodiments

The first through fifteenth embodiments have the following points in common.

The culture container (e.g., the cell-culture container 1) includes the container main body (e.g., the container main body 3) and the filmlike members (e.g., the filmlike members 31, the filmlike members 31A, the filmlike members 31B, the filmlike members 31C, the filmlike members 35, the filmlike members 39, the lower-surface parts 123, and the lower-surface parts 123A). The container main body 3 has the recessed parts (e.g., the recessed parts 7 and the recessed parts 107), wherein the plurality of microwells (e.g., the plurality of microwells 17a and the plurality of microwells 131a) for containing the culture solutions (e.g., the cell-culture solutions C) is formed on the bottom surfaces (e.g., the upper surfaces of the bottom parts 17 and the upper surfaces of the microwell members 131). Each filmlike member is a member that is disposed above the plurality of microwells inside the corresponding recessed part and is for limiting movement of the spheroids (e.g., the spheroids S) grown inside the corresponding microwells such that the spheroids do not separate from the corresponding microwells.

In the present culture container, each filmlike member is disposed above the plurality of microwells inside the corresponding recessed part and limits the movement of the spheroids grown inside those microwells such that the spheroids do not separate from those microwells (e.g., refer to FIG. 8 and FIG. 17). Accordingly, the spheroids are formed stably in the culture container.

The embodiments described above explained the culturing of spheroids, but the present invention is not limited thereto. The present invention can also be adapted to, for example, the culturing of embryonic-layer bodies. An embryoid is a cell mass having a structure similar to that of an early embryo and including ES cells, iPS cells, or the like.

In the embodiments described above, the culture medium is replaced during spheroid formation, but the culture medium does not necessarily have to be replaced.

The shapes of the recessed part and the presser member in plan view as well as combinations thereof are not limited to the embodiments described above.

The number of the recessed parts and the presser members are not limited to the embodiments described above.

The embodiments described above explained, as one example of the culture container, a cell-culture container wherein a cell-culture solution is used. However, the culture container according to the present invention can also culture, for example, animal cells, plant cells, bacteria, and microbes.

### INDUSTRIAL APPLICABILITY

The present invention can be widely adapted to culture containers that are used in observation via a microscope and that contain a culture solution.

### REFERENCE SIGNS LIST

- 1: Cell-culture container
- 3: Container main body
- 5: Presser-member-attached plate
- 5A: Presser-member-attached plate
- 5a: Main body
- 5b: Frame
- 7: Recessed part
- 9: Presser member
- 11: Cover
- 11a: Flat main body
- 11b: Tubular part
- 12: Cushion member
- 15: First engaging part
- 16: Second engaging part
- 17: Bottom part
- 17a: Microwell
- 17b: Mounting hole
- 19: Tubular part
- 19a: Inner circumferential surface
- 25: Tubular part
- 25a: Outer circumferential surface
- 27: Flange part
- 31: Filmlike member
- 31A: Filmlike member
- 31B: Filmlike member
- 31C: Filmlike member
- 31a: Gap
- 31b: Micropore
- 31c: Small hole
- 35: Filmlike member
- 39: Filmlike member
- 39a: Small hole
- 41: Pin member
- 43: Hole
- 45: Projections
- 71: Pin
- 73: Hole
- 103: Container main body
- 107: Recessed part
- 117: Bottom part
- 109: Presser member
- 123: Lower-surface part
- 123A: Lower-surface part
- 123a: Lower surface
- 123B: Ring-shaped protruding part
- 123b: Bubble-discharge part
- 123c: Bubble-discharge part
- 131: Microwell member
- C: Cell-culture solution
- c: Seed cell
- S: Spheroid

## Claims

1. A culture container (1) comprising:
a container main body (3) having a recessed part (7, 107), the recessed part (7, 107) having a bottom part (17, 117, 131) on which a plurality of microwells (17a, 131a) for containing culture solution including a liquid culture medium and seed cells (c) evenly dispersed in the culture medium are formed; and
a filmlike member (31, 31A, 31B, 31C, 35, 39, 123, 123A) disposed above the plurality of microwells (17a, 131a) inside the recessed part (7, 107), the filmlike member (31, 31A, 31B, 31C, 35, 39, 123, 123A) limiting movement of objects grown inside the microwells (17a, 131a) so that the objects do not separate from the microwells (17a, 131a),_ wherein
the filmlike member (31, 31A, 31B, 31C, 35, 39, 123, 123A) has a property or a shape such that a spheroid (S) grown in the microwell (17a, 131a) cannot pass through, **characterized by**
a gap between the filmlike member (31, 31A, 31B, 31C, 35, 39, 123, 123A) and an upper surface of the bottom part (17, 131) has a dimension such that one seed cell (c) can pass through but a spheroid (S) cannot pass through,
wherein the culture container (1) further comprises
a) a presser-member-attached plate (5) including a plate-shaped main body (5a), a presser member (9) that is provided at the main body (5a) and can be inserted into the recessed part (7, 107), and a pin (41) or protrusion (45), wherein the container main body (3) includes a positioning hole (43, 17b), the presser member (9) includes the filmlike member (31, 31A, 31B, 31C, 35, 39, 123, 123A), and a tubular member (25) having a bottom part at which the filmlike member (31, 31A, 31B, 31C, 35, 39, 123, 123A) is provided, in a state that the presser-member-attached plate (5) is fitted to the_container main body (3), the pin (41) or protrusion (45) is inserted into the hole (43, 17b) where a gap between the filmlike member (31, 31A, 31B, 31C, 35, 39, 123, 123A) and the upper surface of the bottom part (17, 131) has a dimension such that one seed cell (c) can pass through but a spheroid (S) cannot pass through; or
b) a presser-member-attached plate (5) including a plate-shaped main body (5a) and a presser member (9, 109) that is provided at the main body and can be inserted into the recessed part (7, 107);
a cover (11) including a flat main body (11a) and a tubular part (11b) extending downward from an outer circumferential edge of the main body (11a), the cover (11) covering the container main body (3) and the presser-member-attached plate (5); and
a cushion member (12) disposed between the presser-member-attached plate (5) and the cover (11), wherein
the presser member (9, 109) includes the filmlike member (31, 31A, 31B, 31C, 35, 39, 123, 123A) and a tubular member (25) having a bottom part at which the filmlike member (31, 31A, 31B, 31C, 35, 39, 123, 123A) is provided,
the presser-member-attached plate (5) is fitted to the container main body where a gap between the filmlike member and an upper surface of the bottom part has a dimension such that one seed cell can pass through but a spheroid cannot pass through, and the cushion member (12) is compressed between the presser-member-attached plate (5) and the cover (11).

2. The culture container (1) according to claim 1, alternative b) wherein
the container main body (3) has an outer circumference part on which a first engagement part (15) is formed,
the tubular part (11b) of the cover (11) is formed with a second engagement part (16), and
the first engagement part (15) and the second engagement part (16) are engaged with each other such that the cushion member (12) is compressed.

3. The culture container (1) according to any of claims 1 through 2, wherein
the filmlike member (123) further includes a bubble-discharge part (123b, 123c).

4. The culture container (1) according to any of claims 1 through 3, further comprising a microwell member (131) provided on a bottom part (117) of the recessed part (107),
the plurality of microwells (131a) are formed on an upper surface of the microwell member (131), and
the bottom part (117) has a surface radially outward of the microwell member (131), the surface being lower than the upper surface of the microwell member (131).

## Patentansprüche

1. Kulturgefäß (1), umfassend:
einen Gefäßhauptkörper (3) mit einem vertieften Bereich (7, 107), wobei der vertiefte Bereich (7, 107) mit einem Bodenbereich (17, 117, 131) versehen ist, auf welchem eine Mehrzahl von Mikrobrunnen (17a, 131a) für die Aufnahme einer Kulturlösung mit einem flüssigen Kulturmedium und in dem Kulturmedium gleichmäßig verteilten Samenzellen (c) gebildet ist; und
ein folienartiges Element (31, 31A, 31B, 31C, 35, 39, 123, 123A), das über den mehrzähligen Mikrobrunnen (17a, 131a) in dem vertieften Bereich (7, 107) angeordnet ist, wobei das folienartige Element (31, 31A, 31B, 31C, 35, 39, 123 123A) die Bewegung von in den Mikrobrunnen (17a, 131a) gewachsenen Objekten derart einschränken, dass sich die Objekte von Mikrobrunnen (17a, 131a) nicht trennen, wobei
das folienartige Element (31, 31A, 31B, 31C, 35, 39, 123, 123A) eine Eigenschaft oder eine Form derart hat, dass ein in dem Mikrobrunnen (17a, 131a) gewachsener Sphäroid (S) nicht hindurchtreten kann,
**dadurch gekennzeichnet, dass**
ein Spalt zwischen dem folienartigen Element (31, 31A, 31B, 31C, 35, 39, 123, 123A) und einer Oberfläche des Bodenbereichs (17, 131) eine Dimension derart hat, dass eine Samenzelle (c) hindurchtreten kann, dass aber ein Sphäroid (S) nicht hindurchtreten kann,
wobei das Kulturgefäß (1) ferner umfasst:
a) eine mit einem Druckelement verbundene Platte (5) mit einem plattenförmigen Hauptkörper (5a), einem Druckelement (9), das an dem Hauptkörper (5a) vorgesehen ist und in den vertieften Bereich (7, 107) eingeführt werden kann, und mit einem Zapfen (41) oder einem Vorsprung (45), wobei der Gefäßhauptkörper (3) eine Positionierungsöffnung (43, 17b) aufweist, wobei das Druckelement (9) das folienartige Element (31, 31A, 31B, 31C, 35, 39, 123, 123A) und ein rohrförmiges Element (25) mit einem Bodenbereich aufweist, an welchem das folienartige Element (31, 31A, 31B, 31C, 35, 39, 123, 123A) vorgesehen ist, in einem Zustand, in dem die mit dem Druckelement verbundene Platte (5) an dem Gefäßhauptkörper (3) montiert ist, der Zapfen (41) oder Vorsprung (45) in die Öffnung (43, 17b) eingesetzt ist, wobei ein Spalt zwischen dem folienartigen Element (31, 31A, 31B, 31C, 35, 39, 123, 123A) und der Oberfläche des Bodenbereichs (17, 131) eine Dimension derart hat, dass eine Samenzelle (c) hindurchtreten kann, dass aber ein Sphäroid (S) nicht hindurchtreten kann; oder
b) eine mit einem Druckelement verbundene Platte (5) mit einem plattenförmigen Hauptkörper (5a) und einem Druckelement (9, 109), das an dem Hauptkörper vorgesehen ist und in den vertieften Bereich (7, 107) eingesetzt werden kann;
eine Abdeckung (11) mit einem flachen Hauptkörper (11a) und einem rohrförmigen Bereich (11b), der sich von einer äußeren Umfangskante des Hauptkörpers (11a) nach unten erstreckt, wobei die Abdeckung (11) den Gefäßhauptkörper (3) und die mit dem Druckelement verbundene Platte (5) abdeckt; und
ein Pufferelement (12), das zwischen der mit dem Druckelement verbundenen Platte (5) und der Abdeckung (11) angeordnet ist, wobei das Druckelement (9, 109) das folienartige Element (31, 31A, 31B, 31C, 35, 39, 123, 123A) und ein rohrförmiges Element (25) mit einem Bodenbereich umfasst, an welchem das folienartige Element (31, 31A, 31B, 31C, 35, 39, 123, 123A) vorgesehen ist, und wobei die mit dem Druckelement verbundene Platte (5) an dem Gefäßhauptkörper montiert ist, wobei ein Spalt zwischen dem folienartigen Element und einer Oberfläche des Bodenbereichs eine Dimension derart aufweist, dass eine Samenzelle hindurchtreten kann, dass aber ein Sphäroid nicht hindurchtreten kann, und wobei das Pufferelement (12) zwischen der mit dem Druckelement verbundenen Platte (5) und der Abdeckung (11) zusammengedrückt wird.

2. Kulturgefäß (1) nach Anspruch 1, Alternative b), wobei der Gefäßhauptkörper (3) einen äußeren Umfangsbereich hat, an welchem ein erster Eingriffsbereich (15) gebildet ist,
wobei der rohrförmige Bereich (11b) der Abdeckung (11) mit einem zweiten Eingriffsbereich (16) ausgebildet ist und
wobei der erste Eingriffsbereich (15) und der zweite Eingriffsbereich (16) sich derart im Eingriff befinden, dass das Pufferelement (12) zusammengedrückt wird.

3. Kulturgefäß (1) nach einem der Ansprüche 1 bis einschließlich 2, wobei das filmähnliche Element (123) ferner einen Blasenaustrittsbereich (123b, 123c) aufweist.

4. Kulturgefäß (1) nach einem der Ansprüche 1 bis einschließlich 3, ferner umfassend ein Mikrobrunnenelement (131), das auf einem Bodenbereich (117) des vertieften Bereichs (107) vorgesehen ist,
wobei die mehrzähligen Mikrobrunnen (131a) auf einer Oberfläche des Mikrobrunnenelements (131) gebildet sind und
wobei der Bodenbereich (117) eine Fläche radial außerhalb des Mikrobrunnenelements (131) hat, die tiefer liegt als die Oberfläche des Mikrobrunnenelements (131).

## Revendications

1. Récipient de culture (1) comprenant :
un corps principal (3) du récipient ayant une partie évidée (7, 107), ladite partie évidée (7, 107) ayant une partie inférieure (17, 117, 131) sur laquelle une pluralité de micropuits (17a, 131a) pour recevoir une solution de culture comprenant un milieu de culture liquide et des cellules germinales (c) réparties uniformément dans ledit milieu de culture sont formés; et
un élément en forme de feuille (31, 31A, 31B, 31C, 35, 39, 123, 123A) disposé au-dessus de la pluralité de micropuits (17a, 131a) dans ladite partie évidée (7, 107), ledit élément en forme de feuille (31, 31A, 31B, 31C, 35, 39, 123 123A) restreignant le mouvement des objets cultivés dans lesdits micropuits (17a, 131a) de sorte que lesdits objets ne sont pas séparés des micropuits (17a, 131a),
l'élément en forme de feuille (31, 31A, 31B, 31C, 35, 39, 123, 123, 123A) ayant une propriété ou une forme telle qu'un sphéroïde (S) cultivé dans les micropuits (17a, 131a) ne peut passer à travers,
**caractérisé en ce qu'**un espace entre ledit élément en forme de feuille (31, 31A, 31B, 31C, 35, 39, 123, 123, 123A) et une surface de ladite partie inférieure (17, 131) a une dimension telle qu'une cellule germinale (c) peut passer à travers mais un sphéroïde (S) ne peut passer à travers,
le récipient de culture (1) comprenant en outre :
a) une plaque (5) reliée à un élément presseur, comprenant un corps principal en forme de plaque (5a), un élément presseur (9) prévu sur le corps principal (5a) et pouvant être inséré dans la partie évidée (7, 107), et une broche (41) ou saillie (45), ledit corps principal (3) du récipient ayant une ouverture de positionnement (43, 17b), ledit élément presseur (9) comprenant ledit élément en forme de feuille (31, 31A, 31B, 31C, 35, 39, 123, 123, 123A) et un élément tubulaire (25) ayant une partie inférieure sur lequel est prévu l'élément en forme de feuille (31, 31A, 31B, 31C, 31C, 35, 39, 123, 123, 123A), dans un état dans lequel la plaque (5) reliée à l'élément presseur est montée sur le corps principal (3) du récipient, la broche (41) ou la saillie (45) est introduite dans l'ouverture (43, 17b), dans laquelle un espace entre l'élément en forme de feuille (31, 31A, 31B, 31C, 35, 39, 123, 123, 123A) et la surface de la partie inférieure (17, 131) a une dimension telle qu'une cellule germinale (c) peut passer à travers mais un sphéroïde (S) ne peut passer à travers; ou
b) une plaque (5) reliée à un élément presseur, comprenant un corps principal en forme de plaque (5a) et un élément presseur (9, 109) prévu sur le corps principal et pouvant être inséré dans la partie évidée (7, 107) ;
un couvercle (11) ayant un corps principal plat (11a) et une partie tubulaire (11b) s'étendant vers le bas à partir d'un bord périphérique extérieur dudit corps principal (11a), ledit couvercle (11) recouvrant ledit corps principal (3) du récipient et ladite plaque (5) reliée audit élément presseur; et
un élément tampon (12) disposé entre la plaque (5) reliée à l'élément presseur et le couvercle (11),
ledit élément presseur (9, 109) comprenant l'élément en forme de feuille (31, 31A, 31B, 31C, 35, 39, 123, 123, 123A) et un élément tubulaire (25) ayant une partie inférieure à laquelle l'élément en forme de feuille (31, 31A, 31B, 31C, 35, 39, 123, 123A) est prévu, et la plaque (5) reliée à l'élément presseur est montée sur le corps principal du récipient, dans lequel un espace entre l'élément en forme de feuille et une surface de la partie inférieure a une dimension telle qu'une cellule germinale peut passer à travers mais un sphéroïde ne peut passer à travers, et dans lequel l'élément tampon (12) est comprimé entre la plaque (5) reliée à l'élément presseur et le couvercle (11).

2. Récipient de culture (1) selon la revendication 1, alternative b), dans lequel le corps principal (3) du récipient présente une région circonférentielle extérieure dans laquelle une première région d'engagement (15) est formée,
la partie tubulaire (11b) du couvercle (11) étant formée avec une seconde partie d'engagement (16) et
dans lequel la première partie d'engagement (15) et la seconde partie d'engagement (16) sont engagées de telle sorte que l'élément tampon (12) est comprimé.

3. Récipient de culture (1) selon l'une quelconque des revendications 1 à 2 inclus, ledit élément en forme de feuille (123) comprenant en outre une région de sortie de bulle (123b, 123c).

4. Récipient de culture (1) selon l'une quelconque des revendications 1 à 3 inclus, comprenant en outre un élément de micropuits (131) prévu sur une partie inférieure (117) de la partie évidée (107),
dans laquelle lesdits puits multiples (131a) sont formés sur une surface dudit élément de micropuits (131), et
la partie inférieure (117) ayant une surface radialement extérieure à l'élément de micropuits (131) qui est plus profonde que la surface de l'élément de micropuits (131).
